## Europäisches Patentamt
## European Patent Office
## Office européen des brevets

(11) Veröffentlichungsnummer: **0 240 714**
**B1**

(12)

# EUROPÄISCHE PATENTSCHRIFT

(45) Veröffentlichungstag der Patentschrift:
06.09.89

(21) Anmeldenummer: 87103021.9

(22) Anmeldetag: 04.03.87

(51) Int. Cl.⁴: **B01J 27/122**, C07C 19/045, C07C 17/156

(54) Geformter Trägerkatalysator und dessen Verwendung bei der Oxichlorierung von Ethylen.

(30) Priorität: 07.03.86 DE 3607449

(43) Veröffentlichungstag der Anmeldung:
14.10.87 Patentblatt 87/42

(45) Bekanntmachung des Hinweises auf die Patenterteilung:
06.09.89 Patentblatt 89/36

(84) Benannte Vertragsstaaten:
BE DE FR GB

(56) Entgegenhaltungen:
DE-A- 3 113 179
DE-A- 3 334 225
GB-A- 1 178 323

(73) Patentinhaber: BASF Aktiengesellschaft,
Carl-Bosch-Strasse 38, D-6700 Ludwigshafen(DE)

(72) Erfinder: Eichhorn, Hans-Dieter, Dr., 47 Davenport Road,
G-B-Cleveland,TS 15 9 TN(GB)
Erfinder: Mross, Wolf Dieter, Dr.,
Anselm-Feuerbach-Strasse 21, D-6710 Frankenthal(DE)
Erfinder: Schachner, Helmut, Dr., Zum Gruenshof 6,
D-6909 Walldorf(DE)
Erfinder: Schwarzmann, Matthias, Dr.,
Carl-Bosch-Strasse 54, D-6703 Limburgerhof(DE)

**Beschreibung**

Die Erfindung betrifft einen geformten Trägerkatalysator, der Kupferionen und Alkaliionen auf einem säulenartigen Träger enthält. Dieser Trägerkatalysator dient insbesondere zur Verwendung bei der Oxichlorierung von Ethylen.

Die Oxichlorierung von Ethylen in einem Katalysatorfestbett ist ein bekanntes, großtechnisch ausgeübtes Verfahren. Die dabei verwendeten Katalysatoren enthalten auf einem Träger aus z.B. Aluminiumoxid Kupfer-(II)-chlorid in Verbindung mit Promotoren wie z.B. Kaliumchlorid. Bei der Durchführung des Verfahrens ist es besonders erwünscht, daß der durch den Katalysator verursachte Druckverlust gering, die wirksame Oberfläche des Katalysators groß und die Wärmeleitfähigkeit zwischen den Katalysatorteilchen und einem gegebenenfalls vorgesehenen inerten Verdünnungsmittel gut ist.

Bei der Verwendung von säulenförmigen Katalysatoren ist der Druckverlust der Katalysatorschüttung in der Regel sehr groß, da die Hohlräume in einem derart gepackten Bett klein sind. Die Erniedrigung des Druckverlustes z.B. durch Vergrößern der Maße bezüglich Durchmesser und Länge der säulenförmigen Katalysatoren führt jedoch nur zu kleineren Umsätzen, da die wirksame Oberfläche des Katalysators dadurch reduziert wird.

Bei der Oxichlorierungsreaktion, bei der sehr viel Wärme frei wird, ist im Katalysatorbett eine gute Wärmeleitfähigkeit erforderlich, da hohe Temperaturspitzen aus Selektivitätsgründen und wegen der Lebensdauer des Katalysators selbst vermieden werden müssen. Aus diesen Gründen treten auch bei der Verwendung von kugelförmigen Katalysatoren Probleme auf, da in diesem Fall die Wärmeleitfähigkeit unzureichend ist, weil die Kontaktfläche zwischen den Katalysatorteilchen vergleichsweise nur gering ist.

Diese genannten Nachteile treten bei der Verwendung von ringförmigen Katalysatoren nicht auf, so daß solche Katalysatorformen besonders vorteilhaft bei der Oxichlorierung von Ethylen verwendet werden. Derartige Katalysatoren sind beispielsweise in der EP-B-0 054 674 oder in der US-A-4 366 093 beschrieben und weisen im allgemeinen einen Durchmesser von 3 bis 12 mm auf. Solche Katalysatorformen werden beispielsweise durch Extrudieren oder Tablettieren hergestellt.

Trotz ihrer vorteilhaften Eigenschaften besteht ein erheblicher Nachteil dieser Katalysatoren darin, daß sie im Vergleich zu den herkömmlichen Säulen- und Kugelformen aufgrund ihrer komplizierten geometrischen Form nur unter sehr hohem Kostenaufwand herzustellen sind.

Die Herstellung solcher ringförmiger Tabletten kann zwar mit einer Tablettierungvorrichtung auf fast die gleiche Weise erfolgen wie die Herstellung einer üblichen massiven säulenförmigen Tablette, jedoch muß die Tablettierform mit einem zentralen Pistill ausgestattet sein, der dem Innendurchmesser des Kreises der herzustellenden zylindrischen Form entspricht. Werden damit jedoch Formen z.B. entsprechend der US-A-4 366 093 hergestellt, so ist aufgrund der kleinen Abmessungen schon bei geringen Verschleißerscheinungen der Tablettierwerkzeuge mit erheblichen Störungen bei der Tablettierung zu rechnen, so daß damit die Herstellungskosten für diesen Formgebungsschritt extrem verteuert werden.

Der Erfindung liegt die Aufgabe zugrunde, einen Katalysator der eingangs genannten Art zur Verfügung zu stellen, der im Vergleich zu den Katalysatoren des Standes der Technik mindestens ebenso vorteilhafte Eigenschaften aufweist, jedoch wesentlich einfacher und preiswerter herzustellen ist.

Diese Aufgabe wird gelöst mit einem geformten Trägerkatalysator, enthaltend Kupferionen und Alkaliionen auf einem säulenartigen Träger, der dadurch gekennzeichnet ist, daß er 2 bis 13 Gew.-% Kupferionen in Form von Kupfer-II-chlorid und/oder Kupferoxichlorid und 0,2 bis 3 Gew.-% Alkalimetallionen enthält und einen Durchmesser von 4 bis 7 mm und eine Höhe, die das 0,35 bis 0,75fache des Durchmessers ausmacht, aufweist.

Die Erfindung betrifft auch die Verwendung des geformten Trägerkatalysators zur Herstellung von 1,2-Dichlorethan durch Oxichlorierung von Ethylen.

Obgleich säulenartige Oxichlorierungskatalysatoren bereits vorgeschlagen wurden und im Vergleich zu den vorteilhafteren Ringkatalysatoren die geschilderten Nachteile bezüglich Aktivität und Druckverlust aufweisen, wurde gefunden, daß sich die Druckverluste reduzieren und Werte entsprechend den vorteilhafteren Ringkatalysatoren annehmen, wenn der Durchmesser der säulenförmigen Katalysatoren im Vergleich zu den Ringkatalysatoren ungefähr um 10 bis 30 % erhöht und die Höhe der säulenförmigen Katalysatoren auf das 0,35 bis 0,75fache des Durchmessers erniedrigt wird. Überraschenderweise werden damit auch die Aktivitäten bzw. die Umsätze erzielt, wie sie mit ringförmigen Katalysatoren erreicht werden. Im Gegensatz zu den ringförmigen Katalysatoren haben die neuen Katalysatoren den Vorteil, daß sie wesentlich einfacher und kostengünstiger herzustellen sind und aufgrund ihrer mechanischen Eigenschaften auch wesentlich besser zu handhaben sind.

Der erfindungsgemäße säulenartig geformte, Kupfer-(II)-chlorid enthaltende Katalysator mit der beschriebenen spezifischen Gestalt und den beschriebenen Abmessungen weist nicht nur einen geringeren Druckverlust, eine große wirksame Oberfläche und eine gute Wärmeleitfähigkeit zwischen den Katalysatorteilchen oder zwischen den Katalysatorteilchen und einem inerten Verdünnungsmittel auf, sondern hat auch eine hervorragende mechanische Festigkeit, die diejenige der ringförmigen Katalysatoren weit übertrifft. Aus diesem Grunde weisen die neuen Katalysatoren auch eine höhere Lebensdauer auf, da bei diesen der Tablettenzerfall, der zum Druckverlustanstieg führt und den Wechsel des Katalysators bedingt, auch viel geringer ist. Der Durchmesser des erfindungsgemäßen säulenartig geformten Katalysatores muß nicht immer konstant sein, d.h. der säulenartige Katalysator kann beispielsweise schwach konisch (sich verjüngend) sein.

Die Verformung der Katalysatoren kann auf übliche Weise unter Benutzung der bekannten Verfahren wie Tablettierung oder Extrudierung erfolgen. Der Verformungsschritt selbst geht zum einen aus von einem Träger oder einem Trägerausgangsmaterial, welches nach der Formgebung, gegebenenfalls nach einem oder mehreren Wärmebehandlungsschritten, mit den Aktivkomponenten getränkt wird. Zum anderen kann der Verformungsschritt auch von einem bereits die Aktivkomponenten enthaltenden pulverförmigen Katalysator ausgehen. Falls es erwünscht wird, kann vor diesem Verformungsschritt auch ein inertes Material mit dem pulverförmigen Katalysator vermischt werden, so daß auf die Weise die Möglichkeit besteht, Katalysatoren mit abgestufter Aktivität herzustellen, wie sie beispielsweise in dem Festbettverfahren in den ersten Reaktorzonen verwendet werden.

Wird die Verformung durch Extrudierverfahren durchgeführt, wird das Ausgangsmaterial in der Regel mit Wasser unter Zusatz von Extrudierhilfsmitteln wie Stärke, Methylcellulose, Graphit, Polyethylenglykolen, Polyvinylalkoholen, Polyvinylpyrrolidonen, Polyacrylester, Stearinsäure und deren Metallsalze, Naphthalin, Ammoniak, Ameisensäure, Oxalsäure oder Salpetersäure entweder als porositätsverbesserndes Mittel oder als Gleitmittel oder als Peptisierungsmittel verformt oder zunächst geknetet und anschließend verformt werden. Bevorzugt ist dabei die Knetung und die anschließende Verformung, insbesondere das Extrudieren.

Die erhaltenen Formlinge werden dann bei Temperaturen im Bereich von Raumtemperatur bis etwa 400°C getrocknet. Anschließend wird bei erhöhten Temperaturen calciniert, wenn die Aktivkomponenten noch nicht aufgebracht sind. Besonders zweckmäßig calciniert man bei einem Temperaturbereich zwischen etwa 500 und 650°C. Daraufhin wird mit den Aktivkomponenten imprägniert.

Wird die Verformung mittels Tablettierverfahren durchgeführt, können dem Ausgangsmaterial ein oder mehrere Zusätze, wie z.B. Bindemittel und/oder Schmiermittel zugesetzt werden. Als Beispiel für solche Zusätze seien Graphit, Stearinsäure und deren Metallsalze wie z.B. Aluminiumstearat oder Magnesiumstearat, Talkum, Methylcellulose bzw. andere modifizierte Cellulosen beispielsweise 1,2-Hydroxymethylcellulose, Glycerinmonostearat, Polyethylenglykole, Polyvinylalkohole, Polyvinylpyrrolidone, Polyacrylester genannt.

Die erhaltenen Formlinge werden dann noch bei höheren Temperaturen bis ca. 650°C calciniert, wenn anschließend noch die Aktivkomponenten auf die Formkörper aufzubringen sind.

Als Trägermaterial können die üblichen Träger wie Kieselgur, Silikagel, Diatomeenerde, Bims, Tonerden, Kieselsäure, Silikate und ähnliches Material verwendet werden. Bevorzugt wird als Trägermaterial Tonerde, insbesondere in Form von $\gamma$-Aluminiumoxid, eingesetzt. Die Trägerpartikel weisen im allgemeinen eine BET-Oberfläche von ca. 30 bis 350 m² auf, ihr Porenvolumen liegt etwa zwischen 0,3 bis 1 cm³/g.

Das Imprägnieren des Trägers mit den katalytisch wirksamen Komponenten kann nach den üblichen Techniken erfolgen. Zur Tränkung des Trägers können also wäßrige oder auch andere, z.B. alkoholische Lösungen, von Chloriden und/oder Oxichloriden des Kupfers verwendet werden.

Im allgemeinen wird der Träger mit wäßrigen Kupfer-(II)-chlorid-Lösungen getränkt. Die Lösung kann noch zusätzlich eine oder mehrere Alkaliverbindungen enthalten, z.B. Kaliumchlorid, Natriumchlorid, Lithiumchlorid, Rubidiumchlorid und/oder Caesiumchlorid, bevorzugt Kaliumchlorid, in einem molekularen Verhältnis von Kupferionen zu Kaliumionen von 1 : 1 bis 10 : 1. Es können aber auch andere Zusätze in der Tränklösung, z.B. Chlorwasserstoff, enthalten sein. Die Konzentration bzw. die Menge an Kupferchlorid-Lösung richtet sich nach der gewünschten Konzentration des Kupfers auf dem Träger.

Nach einer bevorzugten Ausführungsform des erfindungsgemäßen Verfahrens verwendet man 2 bis 10 Gew.-% Kupfer in Form von Kupfer-(II)-chlorid und/oder Kupferoxichlorid. Es ist zweckmäßig, 0,2 bis 3 Gew.-% Alkalimetallionen, insbesondere in Form von Alkalichloriden, zu verwenden. In Betracht kommen sämtliche Alkalimetallionen, insbesondere Chloride, beispielsweise Lithium-, Natrium-, Kalium-, Rubidium- und Caesiumchlorid, besonders bevorzugt Kaliumchlorid.

Das Tränken des Trägers mit der Lösung wird zweckmäßigerweise in einer rotierenden Trommel vorgenommen. Dabei kann entweder der Träger vorgelegt und mit der Lösung besprüht, oder die Lösung vorgelegt und der Träger solange darin bewegt werden, bis die Lösung aufgesaugt ist. Die Temperaturen beim Tränkvorgang sollen vorzugsweise zwischen + 10 und + 70°C liegen.

Das Trocknen des imprägnierten Trägers erfolgt üblicherweise in Luft oder auch in Inertgasatmosphäre wie z.B. Stickstoff bei Temperaturen im Bereich von Raumtemperatur bis etwa 400°C, vorzugsweise durch langsames Erhitzen auf die Trocknungstemperatur und anschließendes mehrstündiges Tempern bei dieser Temperatur, wobei auch eine stufenweise Trocknung von Vorteil sein kann.

Die Durchführung des Oxichlorierungsverfahrens selbst unter Verwendung des erfindungsgemäßen Katalysators kann ein- oder auch mehrstufig erfolgen. Bei mehrstufiger Durchführung können auch einzelne Einsatzstoffe z.B. Luft bzw. Sauerstoff oder Chlorwasserstoff aufgeteilt und einzelnen Stufen getrennt zugeführt werden.

Das aus der Reaktionszone austretende Reaktionsgemisch kann auch bei ein- oder mehrstufiger Fahrweise mit frischem Ethylen, Chlorwasserstoff und Luft bzw. Sauerstoff vermischt in die Reaktionszone zurückgeführt werden. Gegebenenfalls können aus dem Reaktionsgemisch Dichlorethan und Wasser vor der Rückführung vollständig oder zum Teil abgetrennt werden. Die Temperaturen in den Katalysatorschüttungen sollten bei den üblichen Werten zwischen 2OO und 32O°C und die Drücke bei bis etwa 1O bar liegen.

Zur Vermeidung von zu hohen Temperaturspitzen in der Katalysatorschüttung kann es bei der Durchführung des Verfahrens unter Verwendung des erfindungsgemäßen Katalysatorsystems zweckmäßig sein, die Aktivität des Katalysators abzustufen, so daß die Aktivität bei einstufiger Durchführung im Reaktor bzw. bei mehrstufiger Durchführung zumindest in einer Stufe in Produktstromrichtung zunimmt. Die Abstufung der Katalysator aktivität kann durch bekannte Maßnahmen erfolgen, z.B. durch Änderung der Kupfer-(II)-chlorid-Konzentration des Katalysators, durch Dotierung des Katalysators mit Alkali, oder durch Zugabe von Verdünnungsmitteln.

Die Erfindung wird durch die nachstehenden Beispiele noch weiter erläutert:

a) Herstellung der Katalysatoren

Aluminium-metahydroxypulver (Böhmit) wird intensiv mit 3 % Magnesiumstearat vermischt und mit einer Tablettierungsvorrichtung zu Formkörpern mit den folgenden Abmessungen verpreßt:

| a) | Tabletten: | Durchmesser 5 mm, Höhe 5 mm |
|---|---|---|
| b) | Ringtabletten: | Durchmesser außen 5 mm, Höhe 5, mm, Durchmesser innen 2 mm |
| c) | Ringtabletten | Durchmesser außen 7 mm, Höhe 6 mm, Durchmesser innen 3 mm |
| d) | Tabletten: Erfindungsgemäß | Durchmesser 6 mm, Höhe 3 mm |

Nach der Tablettierung werden die Formkörper 3 Minuten bei 55O°C im Luftstrom calciniert und nach Abkühlen mit einem üblichen Imprägnierverfahren mit Kupfer-(II)-chlorid und Kaliumchlorid imprägniert, derart daß die Katalysatoren 7,9 Gew.-% Kupfer und O,89 Gew.-% Kalium aufweisen.

b) Prüfung der Katalysatoren

9O cm³ Katalysator werden in ein Edelstahlrohr mit 64 mm Innendurchmesser eingefüllt und pro Stunde mit einer Gasmischung beaufschlagt, die O,986 mol Chlorwasserstoff, O,493 mol Ethylen und O,246 mol Sauerstoff enthält, wobei Sauerstoff in Form von Luft zugeführt wird. Ein Teil des aus der Katalysatorschüttung austretenden Gases wird mit frischem Einsatzgas vermischt und in die Reaktionszone zurückgeführt. Das Verhältnis von rückgeführtem Reaktionsgas zu frischem Einsatzgas beträgt 2O : 1. Die Temperatur in der Katalysatorschicht beträgt 2OO°C und der Druck 1 bar. Nach Erreichen eines stationären Betriebszustandes werden dem aus der Reaktionszone austretenden Gas Proben entnommen und daraus der Umsatz und die Ausbeute an 1,2-Dichlorethan bestimmt.

Als Maß für die Aktivität der Katalysatoren wird der prozentuale Anteil des umgesetzten Chlorwasserstoffs angegeben.

Zur Prüfung des Druckverlustes werden die entsprechenden Katalysatoren in ein Rohr mit 25 mm innerem Durchmesser eingefüllt, bis sich eine Schütthöhe von einem Meter ergibt. Der Druckverlust der Katalysatorschüttung wird dann bei Raumtemperatur bei einer Beaufschlagung mit 4OOO l Stickstoff pro Stunde gemessen.

Die nachfolgende Tabelle zeigt das Ergebnis der Katalysatorprüfung:

Das Vergleichsbeispiel a) ergibt sowohl einen niedrigeren HCl-Umsatz als auch einen höheren Druckverlust als der erfindungsgemäße Katalysator. Dagegen ergibt sich mit dem Vergleichsbeispiel c) zwar ein niedrigerer Druckverlust jedoch gleichzeitig auch ein wesentlich niedrigerer HCl-Umsatz. Nur das Vergleichsbeispiel b) ergibt ähnlich gute Werte bezüglich des HCl-Umsatzes als auch bezüglich des Druckverlustes wie der erfindungsgemäße Katalysator. Dem Fachmann ist jedoch sofort ersichtlich, daß im Gegensatz zum Vergleichsbeispiel b) der erfindungsgemäße Katalysator wesentlich einfacher und kostengünstiger herzustellen ist und damit erhebliche Vorteile aufweist.

4

Tabelle

| Katalysator | Vergleichsbeispiele | | | Erfindungegemäß |
| --- | --- | --- | --- | --- |
| | Beispiel a) | Beispiel b) | Beispiel c) | Beispiel d) |
| Katalysatorform | Tablette | Ringtablette | Ringtablette | Tablitte |
| Durchmesser, mm | 5 | 5 | 7 | 6 |
| Höhe, mm | 5 | 5 | 6 | 3 |
| Innen-Ø, mm | – | 2 | 3 | – |
| HCl-Umsatz, % | 75,3 | 79,6 | 53,4 | 79,8 |
| Druckverlust, mm Quecksilbersäule | 185 | 94 | 61 | 91 |

## Patentansprüche

1. Geformter Trägerkatalysator, enthaltend Kupferionen und Alkaliionen auf einem säulenartigen Träger, dadurch gekennzeichnet, daß er 2 bis 13 Gew.-% Kupferionen in Form von Kupfer-II-chlorid und/oder Kupferoxichlorid und 0,2 bis 3 Gew.-% Alkalimetallionen enthält und einen Durchmesser von 4 bis 7 mm und eine Höhe, die das 0,35 bis 0,75fache des Durchmessers ausmacht, aufweist.

2. Trägerkatalysator nach Anspruch 1, dadurch gekennzeichnet, daß der Träger einen Durchmesser von 5,5 bis 6,5 mm und eine Höhe von 2,7 bis 3,5 mm aufweist.

3. Trägerkatalysator nach einem der Ansprüche 1 oder 2, dadurch gekennzeichnet, daß er als Alkalimetallionen Kaliumionen enthält.

4. Trägerkatalysator nach einem der Ansprüche 1 bis 3, dadurch gekennzeichnet, daß der Träger aus γ-Aluminiumoxid besteht.

5. Trägerkatalysator nach einem der Ansprüche 1 bis 4, dadurch gekennzeichnet, daß das Porenvolumen des Trägers zwischen 0,3 und 1 cm³/g liegt.

6. Trägerkatalysator nach einem der Ansprüche 1 bis 5, dadurch gekennzeichnet, daß er einen Gehalt an inertem Material aufweist.

7. Trägerkatalysator nach einem der Ansprüche 1 bis 6, erhältlich durch Calcinieren des Trägermaterials bei Temperaturen zwischen 500 und 650°C und anschließendes Imprägnieren des so erhaltenen Trägers mit den Kupfer- und Alkalikomponenten.

8. Verwendung des Trägerkatalysators nach einem der Ansprüche 1 bis 7 zur Herstellung von 1,2-Dichlorethan durch Oxichlorierung von Ethylen.

## Claims

1. A molded supported catalyst containing copper ions and alkali metal ions on a columnar carrier, which contains from 2 to 13% by weight of copper ions in the form of copper(II) chloride and/or copper oxychloride and from 0.2 to 3% by weight of alkali metal ions and has a diameter of from 4 to 7 mm and a height which is from 0.35 to 0.75 times the diameter.

2. A supported catalyst as claimed in claim 1, wherein the carrier has a diameter of from 5.5 to 6.5 mm and a height of from 2.7 to 3.5 mm.

3. A supported catalyst as claimed in claim 1 or 2, wherein the alkali metal ions are potassium ions.

4. A supported catalyst as claimed in any of claims 1 to 3, wherein the carrier comprises γ–Al₂O₃.

5. A supported catalyst as claimed in any of claims 1 to 4, wherein the pore volume of the carrier ranges from 0.3 to 1 cm³/g.

6. A supported catalyst as claimed in any of claims 1 to 5, which contains an inert material.

7. A supported catalyst as claimed in any of claims 1 to 6, obtainable by calcination of the carrier material at from 500 to 600°C and subsequent impregnation of the resulting carrier with copper and alkali metal components.

8. The use of the supported catalyst claimed in one of claims 1 to 7, for preparing 1,2-dichloroethane by oxychlorination of ethylene.

## Revendications

1. Catalyseur façonné, contenant des ions cuivre et des ions alcalins sur un support en forme de colonne, caractérisé en ce qu'il contient de 2 à 13% en poids d'ions cuivre sous forme de chlorure de cuivre-II et/ou d'oxychlorure de cuivre et de 0,2 à 3% en poids d'ions de métal alcalin et il présente un diamètre de 4 à 7 mm et une hauteur qui fait 0,35 à 0,75 fois le diamètre.

2. Catalyseur selon la revendication 1, caractérisé en ce que le support a un diamètre de 5,5 à 6,5 mm et une hauteur de 2,7 à 3,5 mm.

3. Catalyseur selon l'une ou l'autre des revendications 1 et 2, caractérisé en ce qu'il contient des ions potassium en tant qu'ions de métal alcalin.

4. Catalyseur selon l'une des revendications 1 à 3, caractérisé en ce que le support est en oxyde d'aluminium $\gamma$.

5. Catalyseur selon l'une des revendications 1 à 4, caractérisé en ce que le volume de pores du support est entre 0,3 et 1 cm³/g.

6. Catalyseur selon l'une des revendications 1 à 5, caractérisé en ce qu'il présente une teneur en matériau inerte.

7. Catalyseur selon l'une des revendications 1 à 6, obtenu par calcination du support à des températures de 500 à 650°C et imprégnation ultérieure du support ainsi obtenu avec les composés du cuivre et de métal alcalin.

8. Utilisation du catalyseur selon l'une des revendications 1 à 7 pour la préparation de 1,2-dichloro-éthane par oxychloration d'éthylène.